(19) Europäisches Patentamt
European Patent Office
Office européen des brevets

(11) Veröffentlichungsnummer : **0 058 632**
**B1**

# EUROPÄISCHE PATENTSCHRIFT

(45) Veröffentlichungstag der Patentschrift : **11.04.84**

(21) Anmeldenummer : **82730013.8**

(22) Anmeldetag : **12.02.82**

(51) Int. Cl.³ : **C 07 C177/00**, A 61 K 31/557// C07C69/593, C07C69/533, C07C57/13, C07C57/03, C07F9/40, C07F7/18

(54) **(13E)-(8R,11R,12R)-11,15-Dihydroxy-16,19-dimethyl-9-oxo-13,18-prostadiensäuren sowie deren Salze, Verfahren zu ihrer Herstellung und diese enthaltende pharmazeutische Zusammensetzungen.**

(30) Priorität : **13.02.81 DE 3106149**
**22.01.82 DE 3202457**

(43) Veröffentlichungstag der Anmeldung : **25.08.82 Patentblatt 82/34**

(45) Bekanntmachung des Hinweises auf die Patenterteilung : **11.04.84 Patentblatt 84/15**

(84) Benannte Vertragsstaaten : **AT BE CH DE FR GB IT LI LU NL SE**

(56) Entgegenhaltungen : **DE-A- 2 635 985**

(73) Patentinhaber : **SCHERING AKTIENGESELLSCHAFT**
**Berlin und Bergkamen**
**Müllerstrasse 170/178 Postfach 65 03 11**
**D-1000 Berlin 65 (DE)**

(72) Erfinder : **Vorbrüggen, Helmut, Prof. Dr.**
**Wilkestrasse 7**
**D-1000 Berlin 27 (DE)**
Erfinder : **Schwarz, Norbert, Dr.**
**Stubenrauchstrasse 31**
**D-1000 Berlin 41 (DE)**
Erfinder : **Elger, Walter, Dr.**
**Schorlemer Allee 12b**
**D-1000 Berlin 33 (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

EP 0 058 632 B1

(13E)-(8R,11R,12R)-11,15-Dihydroxy-16,19-dimethyl-9-oxo-13,18-prostadiensäuren sowie deren Salze, Verfahren zu ihrer Herstellung und diese enthaltende pharmazeutische Zusammensetzungen

Die vorliegende Erfindung betrifft (13E)-(8R,11R,12R)-11,15-Dihydroxy-16,19-dimethyl-9-oxo-13,18-prostadiensäuren, deren physiologisch verträglichen Salze, Verfahren zu ihrer Herstellung und diese enthaltende pharmazeutische Zusammensetzungen.

In der deutschen Offenlegungsschrift 26 35 985 werden Prostansäurederivate der allgemeinen Formel

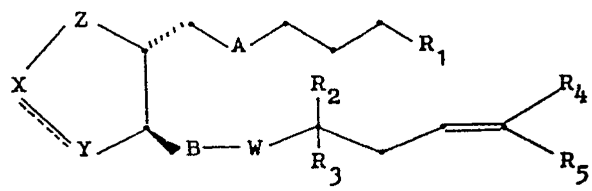

beansprucht, in der

$R_1$ eine $-\underset{\underset{O}{\|}}{C}-R_6$ oder $-\underset{R_7\ R_8}{\overset{}{C}}-OR_9$-Gruppe darstellt, worin $R_6$ für eine Hydroxylgruppe, eine gerade oder verzweigte Alkoxygruppe mit 1-10 C-Atomen, eine substituierte oder unsubstituierte Aryloxygruppe, eine $O-CH_2-U-V$-Gruppe, wobei U eine direkte Bindung, eine Carbonyl- oder Carbonyloxygruppe und V einen durch eine oder mehrere Phenylgruppen, Alkoxygruppen mit 1-2 C-Atomen oder Halogenatome, vorzugsweise Bromatome, substituierten Phenylring bedeutet oder für eine $-NHR_{10}$-Gruppe steht, worin $R_{10}$ eine Alkyl- oder Arylgruppe oder einen Säurerest einer organischen Carbon- oder Sulfonsäure mit 1-15 C-Atomen darstellt, $R_7$ und $R_8$ Wasserstoffatome oder Alkylgruppen mit 1-4 C-Atomen und $R_9$ entweder einen Säurerest einer organischen Carbon- oder Sulfonsäure mit 1-15 C-Atomen oder einer anorganischen Säure oder eine $-\underset{\underset{O}{\|}}{C}-NHR_{10}$-Gruppe darstellen, wobei $R_{10}$ die oben angegebene Bedeutung besitzt,

A eine $-CH_2-CH_2$- oder eine cis- oder trans-CH=CH-Gruppe bedeutet,

B eine $-CH_2-CH_2$-, eine trans-CH=CH-, eine $-C\equiv C$-Gruppe oder eine $-\underset{\diagdown CH_2 \diagup}{CH-CG}$-Gruppe, wobei die Methylengruppe α- oder β-ständig sein kann, bedeutet,

W eine freie oder funktionell abgewandelte Hydroxymethylengruppe, eine freie oder funktionell abgewandelte Carbonyl- oder eine $-\overset{R_{11}}{\underset{OH}{C}}-$-Gruppe, wobei $R_{11}$ ein Wasserstoffatom oder eine Alkylgruppe mit 1-5 C-Atomen und die OH-Gruppe α- oder β-ständig und funktionell abgewandelt sein kann, bedeutet,

Z eine Carbonyl- oder Hydroxymethylengruppe, die frei oder funktionell abgewandelt sein können, darstellt,

X ... Y für $-CH_2-\underset{R_{12}}{CH}$- oder $-CH_2-\underset{\underset{O}{\|}}{C}$- steht, wenn Z eine freie oder funktionell abgewandelte Hydroxymethylengruppe bedeutet oder für $-CH_2-\underset{R_{12}}{CH}$- oder $-CH=CH$-, wenn Z eine freie oder funktionell abgewandelte Carbonylgruppe darstellt, wobei der Rest $R_{12}$ ein Wasserstoffatom, eine Methyl-, eine Cyanidgruppe oder ein freie oder funktionell abgewandelte Hydroxygruppe bedeutet,

$R_2$ für ein Wasserstoffatom oder eine Alkylgruppe steht,

$R_3$ ein Wasserstoffatom oder eine Alkylgruppe bedeutet,

$R_4 = R_5$ für eine Methylgruppe steht oder

$R_4$ für ein Chloratom steht, wenn $R_5$ eine Methylgruppe bedeutet oder

$R_5$ für ein Chloratom steht, wenn $R_4$ eine Methylgruppe bedeutet,

und, falls $R_6$ eine Hydroxygruppe darstellt, deren physiologisch verträgliche Salze mit Basen bedeuten.

Die Verbindungen besitzen wertvolle therapeutische Eigenschaften und zeichnen sich insbesondere dadurch aus, daß sie nach einmaliger intrauteriner Applikation eine Menstruation zu induzieren oder eine Schwangerschaft zu unterbrechen vermögen. Sie eignen sich ferner zu Synchronisation des Sexualcyclus bei weiblichen Säugetieren wie Affen, Rindern, Schweinen, Schafen usw. Die Prostaglandinderivate aus der DE-OS 26 35 985 wirken stark uteruskontrahierend sowie luteolytisch, d. h. zur Auslösung eines Aborts benötigt man geringere Dosierungen als bei den entsprechenden natürlichen Prostaglandinen. Bei den Prostaglandinen sind die gewünschten Hauptwirkungen meistens von unerwünschten Nebenwirkungen begleitet, die die Qualität der Hauptwirkung wesentlich herabsetzen.

Unter den in der DE-OS 26 35 985 beanspruchten Verbindungen zeigten die (13E)-(8R,11R,12R)-11,15-Dihydroxy-16,19-dimethyl-9-oxo-13,18-prostadiensäuren der allgemeinen Formel I

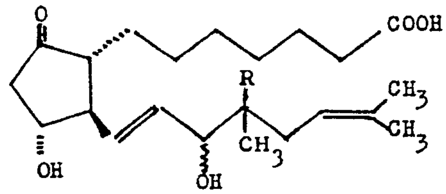

(I)

worin

R Wasserstoff oder eine Methylgruppe bedeutet oder ihre physiologisch verträglichen Salze mit Basen als Abortiva derartig herausragende Eigenschaften, daß damit die Dosierung gegenüber handelsüblichen Präparaten (Sulproston) um ein Vielfaches herabgesetzt werden kann, wodurch natürlich auch unerwünschte Nebenwirkungen noch stärker zurückgedrängt werden. Gegenüber den Verbindungen in DE-OS 26 35 985 zeigen die ausgewählten Verbindungen eine relative Wirksamkeit von mindestens 10-100. Die (13E)-(8R,11R,12R)-11,15-Dihydroxy-16,19-dimethyl-9-oxo-13,18-prostadiensäuren sind in der DE-OS 26 35 985 nicht namentlich beschrieben. Verbindungen mit A als $—CH_2—CH_2$-Gruppe werden gegenüber den anderen Verbindungen, in denen A eine cis-CH=CH-Gruppe bedeutet, nicht herausgestellt.

Die Erfindung betrifft auch ein Verfahren zur Herstellung von Verbindungen der Formel I, dadurch gekennzeichnet, daß man (8R,9S,11R,12S)-9-Benzoyloxy-13-oxo-11-(tetrahydropyran-2-yloxy)-14,15,16,17,18,19,20-heptanorprostansäuremethylester mit 2-(1,4-Dimethyl-3-pentenyl)-2-oxoäthanphosphonsäuredimethylestern der Formel II

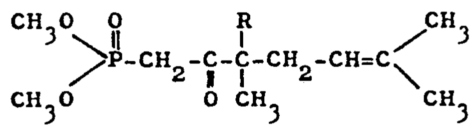

(II)

worin

R die oben angegebene Bedeutung hat, umsetzt und anschließend die 15-Ketogruppe reduziert und mit Dihydropyran schützt, die 9-Hydroxygruppe nach Freisetzung oxidiert, vorhandene Schutzgruppen abspaltet, wenn R Wasserstoff bedeutet, in die Stereoisomeren trennt und gegebenenfalls in ein physiologisch verträgliches Salz überführt.

Die Herstellung der 2-(1,4-Dimethyl-3-pentenyl)-2-oxoäthanphosphonsäuredimethylester der Formel II erfolgt nach an sich bekannten Verfahren, wie sie in der DE-OS 26 35 985 beschrieben worden sind.

Zur Salzbildung kommen alle anorganischen und organischen Basen in Frage, wie sie dem Fachmann zur Bildung physiologisch verträglicher Salze bekannt sind. Beispielsweise seien genannt Alkalihydroxide, wie Natrium- oder Kaliumhydroxid, Erdalkalihydroxide, wie Calciumhydroxid, Ammoniak, Amine, wie Äthanolamin, Diäthanolamin, Triäthanolamin, N-Methylglucamin, Morpholin, Tris-(hydroxymethyl)-methylamin usw.

Die Umsetzung von (8R,9S,11R,12S)-9-Benzoyloxy-13-oxo-11-(tetrahydropyran-2-yloxy)-14,15,16,17,18,19,20-heptanor-prostansäuremethylester mit Verbindungen der Formel II wird in an sich bekannter Weise bei Temperaturen von 0 °C bis 100 °C, vorzugsweise bei 20 °C bis 80 °C, in einem aprotischen Lösungsmittel durchgeführt, wie beispielsweise Dimethylsulfoxid, Dimethylformamid, Benzol, Toluol, Xylol, Diäthyläther, Tetrahydrofuran, Dioxan, Chloroform, Methylenchlorid, Dimethoxyäthan, usw.

Die Oxidation der 9-Hydroxygruppe wird nach an sich bekannten Methoden mit den üblichen Oxidationsmitteln vorgenommen. Beispielsweise kann die Oxidation unter intermediärem Schutz der 11- und 15-Hydroxygruppen, z. B. durch Silylierung (Chem. Comm. *1972*, 1120) mit Jones-Reagenz erfolgen.

Die Freisetzung der funktionell abgewandelten Hydroxygruppen erfolgt nach bekannten Methoden. Beispielsweise wird die Abspaltung von Hydroxyschutzgruppen, wie beispielsweise des Tetrahydropyranylrestes, in einer wäßrigen Lösung einer organischen Säure, wie zum Beispiel Oxalsäure, Essigsäure, Propionsäure u. a., oder in einer wäßrigen Lösung einer anorganischen Säure, wie zum Beispiel Salzsäure, durchgeführt. Zur Verbesserung der Löslichkeit wird zweckmäßigerweise ein mit Wasser mischbares inertes organisches Lösungsmittel zugesetzt. Geeignete organische Lösungsmittel sind zum Beispiel Alkohole, wie Methanol und Äthanol, Äther, wie Dimethoxyäthan, Dioxan und Tetrahydrofuran und Aceton. Tetrahydrofuran wird bevorzugt angewendet. Die Abspaltung wird vorzugsweise bei Temperaturen zwischen 20 °C und 80 °C durchgeführt.

Die Verseifung der Acylgruppen erfolgt beispielsweise mit Alkali- oder Erdalkali-carbonaten oder -hydroxyden in einem Alkohol oder in der wäßrigen Lösung eines Alkohols. Als Alkohole kommen

aliphatische Alkohole in Betracht, wie zum Beispiel Methanol, Äthanol, Butanol usw., vorzugsweise Methanol. Als Alkalicarbonate und -hydroxyde seien Kalium- und Natriumsalze genannt, bevorzugt sind die Kaliumsalze. Als Erdalkalicarbonate und -hydroxyde sind beispielsweise geeignet Calciumcarbonat, Calciumhydroxyd und Bariumcarbonat, Die Umsetzung erfolgt bei – 10 °C bis + 70 °C, vorzugsweise bei + 25 °C.

Die (13E)-(8R,11R,12R)-11,15-Dihydroxy-16,19-dimethyl-9-oxo-13,18-prostadiensäuren können mit geeigneten Mengen der entsprechenden anorganischen Basen unter Neutralisierung in Salze überführt werden. Beispielsweise erhält man beim Lösen der PG-Säure in Wasser, welches die stöchiometrische Menge der Base enthält, nach Abdampfen des Wassers oder nach Zugabe eines mit Wasser mischbaren Lösungsmittels, zum Beispiel Alkohol oder Aceton, das feste anorganische Salz.

Zur Herstellung eines Aminsalzes, die in üblicher Weise erfolgt, wird die PG-Säure zum Beispiel in einem geeigneten Lösungsmittel, beispielsweise Äthanol, Aceton, Diäthyläther oder Benzol gelöst und mindestens die stöchiometrische Menge des Amins dieser Lösung zugesetzt. Dabei fällt das Salz gewöhnlich in fester Form an oder wird nach Verdampfen des Lösungsmittels in üblicher Weise isoliert.

Die gute Wirkungsdissoziation der erfindungsgemäßen Substanz zeigt sich bei der Untersuchung an anderen glattmuskulären Organen, wie beispielsweise am Meerschweinchen-Ileum oder an der isolierten Kaninchen-Trachea, wo eine wesentlich geringere Stimulierung zu beobachten ist als durch die natürlichen Prostaglandine.

Der erfindungsgemäße Wirkstoff zeigt an der isolierten Kaninchen-Trachea in vitro bronchodilatorische Wirkung und hemmt stark die Magensäuresekretion und wirkt regulierend bei Herzrhythmusstörungen. Die neue Verbindung senkt ferner den Blutdruck und wirkt diuretisch.

Für die medizinische Anwendung kann der Wirkstoff in eine für die Inhalation, für orale oder parenterale Applikation geeignete Form überführt werden.

Zur Inhalation werden zweckmäßigerweise Aerosol- oder Spraylösungen hergestellt.

Für die orale Applikation sind beispielsweise Tabletten, Dragees oder Kapseln geeignet.

Für die parenterale Verabreichung werden sterile, injizierbare, wäßrige oder ölige Lösungen benutzt. Die Einheitsdosis liegt je nach Anwendung (vaginal, extraamnial, intramuskulär, intravenös) zwischen 0,25 µg und 50 mg.

Die Erfindung betrifft damit auch Arzneimittel auf Basis der Verbindungen in Anspruch 1 und üblicher Hilfs- und Trägerstoffe.

Der erfindungsgemäße Wirkstoff soll in Verbindung mit den in der Galenik bekannten und üblichen Hilfsstoffen, zum Beispiel zur Herstellung von Präparaten zur Auslösung eines Abortes, zur Zyklussteuerung oder zur Einleitung einer Geburt dienen. Für diesen Zweck können sterile, wäßrige Lösungen, die 0,000 1-10 µg/ml der aktiven Verbindung enthalten, als intravenöse Infusionslösung angewendet werden. Zur Herstellung wäßriger isotonischer Lösungen sind die Säure und ihre Salze besonders geeignet. Zur Steigerung der Löslichkeit können Alkohole, wie Äthanol und Propylenglykol, hinzugefügt werden. Ferner lassen sich leicht Suppositorien zur intravaginalen Applikation herstellen.

## Beispiel 1

(13E)-(8R,11R,12R,15S,16RS)-11,15-Dihydroxy-16,19-dimethyl-9-oxo-13,18-prostadiensäure

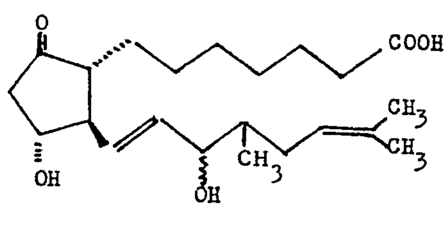

a) 2-Äthoxycarbonyl-2,5-dimethyl-4-hexensäureethylester

In einem mit Rückflusskühler, Tropftrichter und Rührer versehenen Dreihalskolben wurden 36,1 g Natrium (in kleine Stücke geschnitten) gegeben. Hierzu wurden 800 ml abs. Äthanol so schnell zugetropft, dass die Lösung in lebhaftem Sieden blieb. Zur heissen Alkoholat-Lösung tropfte man 269,6 g frisch destillierten Methylmalonsäurediäthylester, rührte 1/2 Stunde bei 60 °C und gab dann-ebenfalls tropfenweise 241,7 g Dimethylallylbromid hinzu. Nach einer Stunde Rühren unter Erhitzen filtrierte man das ausgefallene Natriumbromid ab, wusch den Niederschlag und engte das Filtrat ein. Der Rückstand wurde in Äther aufgenommen, mit gesättigter Natriumchlorid-Lösung neutral gewaschen, über Magnesiumsulfat getrocknet und am Rotationsverdampfer eingeengt. Der Eindampfrückstand wurde an der Ölpumpe fraktioniert. Man erhielt 266 g der Titelverbindung vom $Kp_7$ = 97-112 °C.

IR (Film) : 1 735, 1 245, 1 025, 860/cm.

b) 2-Carboxy-2,5-dimethyl-4-hexensäure

223,8 g des nach a) erhaltenen Diesters wurden zusammen mit 181 g Kaliumhydroxid in 235 ml Wasser und 450 ml Äthanol 4 Stunden unter Rückfluss erhitzt. Anschliessend wurde das Äthanol am Rotationsverdampfer abgezogen, der Rückstand in 235 ml Wasser gelöst und unter Eiskühlung mit konzentrierter Salzsäure bis zum pH-Wert 1 tropfenweise versetzt. Der Niederschlag (Fp 162-166 °C) wurde gesammelt, mit Wasser gewaschen und ohne weitere Reinigung in die nächste Stufe eingesetzt.

IR (KBr) 1 700, 1 230, 840/cm.

c) 2,5-Dimethyl-4-hexensäure

Die in der vorigen Reaktionsstufe erhaltene Dicarbonsäure wurde in einer Destillationsapparatur 4 Stunden bei Normaldruck, anschliessend eine Stunde bei 75 Torr auf 210 °C gehalten. Das Produkt wurde dann im Vakuum destilliert. Es wurden 68 g der Titelverbindung ($Kp_5$ = 98-106 °C ; $Kp_1$ = 67-70 °C) erhalten.

Ir (Film) : 1 705, 1 220, 810/cm.

d) 2,5-Dimethyl-4-hexensäure-methylester

Die nach obiger Vorschrift erhaltenen 68 g Carbonsäure wurden mit so viel ätherischer Diazomethan-Lösung versetzt, bis sich kein Stickstoff mehr bei Zugabe des Reagenzes entwickelte und die Gelbfärbung der Reaktionslösung bestehen blieb. Das Lösungsmittel wurde dann im Vakuum entfernt und der Rückstand fraktioniert, Man erhielt 62,3 g von $Kp_{3,5-6}$ = 32-35 °C.

IR (Film) : 1 735, 1 160, 1 050, 820/cm.

e) 2,5-Dimethyl-4-hexensäure-äthylester

85,3 g 2-Äthoxycarbonyl-2,5-dimethyl-4-hexensäureäthylester wurden in 645 ml Dimethylsulfoxid gelöst und nacheinander mit 29,7 g Lithiumchlorid und 6,3 ml destilliertem Wasser versetzt. Danach wurde das Reaktionsgemisch insgesamt 13 Stunden auf 200 °C erhitzt und anschliessend — nach Abkühlen lassen — auf 1 l Eiswasser gegossen. Die wässrige Phase wurde dreimal mit je 500 ml Methylenchlorid extrahiert. Die vereinigten organischen Extrakte wurden dann zweimal mit Wasser gewaschen, über Magnesiumsulfat getrocknet, am Rotationsverdampfer eingeengt und im Vakuum destilliert. Man isolierte 53,1 g vom $Kp_{13}$ = 75-78 °C.

IR (Film) : 1 735, 1 160, 1 050/cm.

f) 2-(1,4-Dimethyl-3-pentenyl)-2-oxoäthanphosphonsäure-dimethylester

Zu einer Lösung von 59 g Methanphosphonsäuredimethylester in 400 ml abs. Tetrahydrofuran tropfte man unter Argon bei – 60 °C 274,7 ml eine 1,61 m Butyllithium-Lösung in Hexan. Nach 15 Minuten Nachrühren wurde eine Lösung von 34,05 g 2,5-Dimethyl-4-hexensäure-äthylester in 100 ml abs. Tetrahydrofuran zugetropft. Man liess das Reaktionsgemisch innerhalb von vier Stunden sich auf Raumtemperatur erwärmen und rührte es anschliessend noch 3 Stunden. Danach wurde es mit 26,5 ml Eisessig versetzt und im Vakuum eingeengt. Der Rückstand wurde mit Äther/Wasser aufgenommen, die wässrige Phase mit festem Natriumchlorid versetzt und ausgeäthert. Die vereinigten organischen Phasen wurden über Magnesiumsulfat getrocknet und am Rotationsverdampfer eingeengt. Der Verdampfungsrückstand wurde durch Säulenchromatographie an Kieselgel mit Hexan/50-100 % Essigester als Fliessmittel gereinigt. Man erhielt 32 g der gewünschten Verbindung.

IR (Film) : 1 710, 1 260, 1 030/cm.

g) (1S,5R,6S,7R)-6-[(tert.-Butyl-dimethyl-silyloxy)-methyl]-7-benzoyloxy-2-oxabicyclo [3.3.0] octan-3-on

Zu einer Lösung von 13,8 g (1S,5R,6R,7R)-6-Hydroxymethyl-7-benzoyloxy-2-oxabicyclo (3.3.0) octan-3-on in 30 ml abs. Dimethylformamid wurden eine Lösung von 9,9 g Dimethyl-tert.-butyl-chlorsilan in 40 ml abs. Dimethylformamid und 9,35 g Imidazol gegeben. Nach zweistündigem Rühren bei Raumtemperatur und unter einer Argon-Atmosphäre zeigte die analytische Dünnschichtchromatographie vollständige Umsetzung an. Das Reaktionsgemisch wurde mit 850 ml Äther verdünnt, mit ca. 60 ml gesättigter Natriumbicarbonat-Lösung und gesättigter Natriumchlorid-Lösung gewaschen und anschliessend über Magnesiumsulfat getrocknet. Der Eindampfrückstand kann gegebenenfalls durch Säulenchromatographie an Kieselgel mit Äther als Fliessmittel gereinigt werden. Man erhielt 17,8 g der Titelverbindung (Fp = 74-75 °C nach Kristallisation aus Pentan/Äther).

IR : 1 775, 1 715, 1 600, 1 580, 1 275, 1 255, 840, 790, 720/cm.

h) (1S,5R,6S,7R)-6-[(tert.-Butyl-dimethylsilyloxy)-methyl]-7-hydroxy-2-oxabicyclo [3.3.0] octan-3-on

Eine Lösung von 17,3 g des in der vorigen Reaktionsstufe erhaltenen Benzoats in 200 ml abs.

Methanol wurde bei Raumtemperatur zusammen mit 6,5 g trockenem Kaliumcarbonat unter Argon gerührt. Nach zwei Stunden zeigte die analytische Dünnschicht-Chromatographie vollständige Umsetzung an. Man tropfte daraufhin bei 0 °C 500 ml 0,1 n Salzsäure zum Reaktionsgemisch, rührte 15 Minuten bei Raumtemperatur nach, engte im Vakuum ein und extrahierte mit Essigester. Die organische Phase wurde mit gesättigter Natriumchlorid-Lösung gewaschen und über Magnesiumsulfat getrocknet. Der Eindampfungsrückstand wurde durch Säulenchromatographie an Kieselgel mit Hexan/50-100 % Äther als Fliessmittel gereinigt. Es wurden 9,1 g der gewünschten Verbindung (Fp. : 57-58,5 °C) erhalten.

IR (Film) : 1 775, 1 255, 840, 790/cm.

i) (1S,5R,6S,7R)-6-[(tert.-Butyl-dimethylsilyloxy)-methyl]-7-(tetrahydropyran-2-yloxy)-2-oxabicyclo [3.3.0] octan-3-on

Eine Lösung von 15,8 g des nach obiger Vorschrift erhaltenen Alkohols in 300 ml destilliertem Methylenchlorid wurde zusammen mit 7,5 ml frisch über Kaliumhydroxid destilliertem Dihydropyran und 1,38 g Pyridin-p-Toluolsulfonat 14 Stunden bei Raumtemperatur gerührt. Nach dem Einengen der Reaktionslösung bei Raumtemperatur wurde mit Äther verdünnt und mit halbgesättigter Natriumchlorid-Lösung gewaschen. Die organische Lösung wurde anschliessend über Magnesiumsulfat getrocknet und dann zur Trockne eingeengt. Gegebenenfalls kann das Produkt durch Säulenchromatographie an Kieselgel mit Hexan/20-50 % Äther als Fliessmittel gereinigt werden. Die Ausbeute betrug 20 g.

IR (Film) : 1 775, 1 255, 1 115, 1 080, 1 035, 835, 775/cm.

j) (1S,3RS,5R,6S,7R)-3-Hydroxy-6-[(tert.-Butyl-dimethylsilyloxy)-methyl]-7-(tetrahydropyran-2-yloxy)-2-oxalicyclo [3.3.0] octan

Zu einer auf − 70 °C gekühlten Lösung von 5,4 g des in der vorigen Reaktionsstufe erhaltenen Lactons in 200 ml abs. Toluol wurden unter Argon innerhalb von 15 Minuten 20 ml einer 20 %igen DIBAH-Lösung in Toluol zugetropft. Nach ca. 5 minütigem Nachrühren wurden bei der gleichen Temperatur 1,2 ml Isopropanol zugetropft bis keine Schaumbildung mehr auftrat. Man liess die Reaktionslösung sich auf 0 °C erwärmen, gab 16 ml Wasser dazu, rührte 10 Minuten nach und filtrierte über eine Fritte ab. Der Niederschlag wurde mit Essigester ausgewaschen. Die organische Phase wurde dreimal mit gesättigter Natriumchlorid-Lösung gewaschen, über Magnesiumsulfat getrocknet und am Rotationsverdampfer eingeengt. Das erhaltene Produkt (5,41 g) wurde ohne weitere Reinigung in die nächste Reaktionsstufe eingesetzt.

k) (5Z)-(8R,9S,11R,12S)-9-Hydroxy-11-(tetrahydropyran-2-yloxy)-13-(tert.-butyl-dimethyl-silyloxy)-14, 15,16,17,18,19,20-heptanor-5-prostensäure

104,6 ml einer Lösung von Methansulfinylmethylnatrium in abs. DMSO (hergestellt durch Lösen von 6 g 50 %iger Natriumhydridsuspension in 120 ml abs. Dimethylsulfoxid bei maximal 70 °C) wurden bei 15 °C zu einer Lösung von 25,67 g 4-Carboxybutyl-triphenylphosphoniumbromid (vorher bei 70-80 °C an der Ölpumpe getrocknet) in 80 ml abs. Dimethylsulfoxid getropft. Diese Ylenlösung wurde 30 Minuten bei Raumtemperatur gerührt und dann unter Wasserkühlung zu einer Lösung von 5,41 g des in der vorigen Reaktionsstufe erhaltenen Lactols in 50 ml abs. Dimethylsulfoxid innerhalb von 15 Minuten getropft. Anschliessend wurde das Reaktionsgemisch vier Stunden bei 35-40 °C unter Argon gerührt. (Gegebenenfalls setzt man 50-100 ml abs. Tetrahydrofuran zur Reaktionslösung zu. Zur Aufarbeitung wurde das Reaktionsgemisch auf Eis/Wasser gegeben, dreimal mit Äther extrahiert, die wässrige Phase mit 10 %iger Citronensäurelösung bis pH = 4 angesäuert und dreimal mit einem 1/1-Gemisch aus Äther/Hexan extrahiert. Danach wurde noch dreimal mit Methylenchlorid ausgeschüttelt. Aufgrund der analytischen Dünnschichtchromatographie wurde die Methylenchlorid-Phase verworfen, die beiden anderen organischen Phasen dagegen vereinigt, über Magnesiumsulfat getrocknet, filtriert und am Rotationsverdampfer eingeengt. Der Rückstand wurde durch Säulenchromatographie an Kieselgel mit Hexan/70-100 % Äther als Fliessmittel gereinigt.

Man erhielt 4,32 g der Carbonsäure.

IR (Film) : 3 440 (breit), 3 200-2 500, 1 725, 1 700, 1 250, 1 100, 1 020, 830, 770/cm.

l) (5Z)-(8R,9S,11R,12S)-9-Hydroxy-11-(tetrahydropyran-2-yloxy)-13-(tert.-butyl-dimethyl-silyloxy)-14, 15,16,17,18,19,20-heptanor-5-prostensäure-methylester

Darstellung mit Diazomethan :

Die nach k) erhaltenen 4,32 g Carbonsäure wurden in 20 ml Methylenchlorid gelöst und so lange mit ätherischer Diazomethanlösung versetzt, bis keine Gasentwicklung mehr auftrat und die Gelbfärbung der Lösung bestehen blieb. Nach Abziehen des überschüssigen Diazomethans am Wasserstrahlvakuum wurde die Reaktionslösung am Rotationsverdampfer zur Trockne eingeengt. Man erhielt 4,3 g des gewünschten Carbonsäuremethylesters.

Darstellung mit Jodmethan :

Zu einer Lösung von 32,5 g der nach k) erhaltenen Carbonsäure in 450 ml Acetonitril wurde bei Raumtemperatur 75 ml N-Äthyldiisopropylamin und 150 ml Jodmethan in 200 ml Acetonitril innerhalb von 2 Stunden zugetropft. Es wurde eine Stunde nachgerührt, dann — nach erfolgter DC-Kontrolle — saugte man den Niederschlag ab, wusch mit Essigester nach und schüttelte die organische Phase nacheinander mit Natriumbisulfat-, Natrium-hydrogencarbonat- und Natriumchlorid-Lösung. Nach Trocknen über Magnesiumsulfat wurde am Rotationsverdampfer zur Trockne eingeengt und der Rückstand durch Säulenchromatographie an Kieselgel mit Hexan/50-100 % Äther als Laufmittel gereinigt. Man erhielt 32,3 g der Titelverbindung.

IR (Film) : 3 420 (breit), 1 740, 1 255, 1 120, 1 030, 840, 780/cm.

m) (5Z)-(8R,9S,11R,12S)-9-Benzoyloxy-11-(Tetrahydropyran-2-yloxy)-13-(tert.-butyl-dimethylsilyloxy)-14,15,16,17,18,19,20-heptanor-5-prostensäuremethylester

Zu einer Lösung von 4,7 g der nach 1) erhaltenen 9-Hydroxy-Verbindung in 70 ml Pyridin wurden unter Rühren bei Raumtemperatur 2,32 ml destilliertes Benzoylchlorid getropft. Nach zweistündigem Rühren unter einer Argon-Atmosphäre wurde die Reaktionslösung mit 1,8 ml Wasser versetzt, eine weitere Stunde nachgerührt und anschliessend an der Ölpumpe bei 30 °C und 1 Torr eingeengt. Der Rückstand wurde in einem Zweiphasengemisch Äther/Wasser aufgenommen, mit Natriumbicarbonat- und gesättigter Natriumchlorid-Lösung gewaschen, über Magnesiumsulfat getrocknet und im Vakuum zur Trockne eingeengt. Nach Säulenchromatographie des Eindampfrückstands an Kieselgel mit Hexan/30-50 % Essigester als Fliessmittel erhielt man 5,11 g der Titelverbindung als farbloses Öl.

IR (Film) : 1 745, 1 720, 1 605, 1 590, 1 280, 1 260, 1 120, 1 030, 840, 780, 710/cm.

n) (8R,9S,11R,12S)-9-Benzoyloxy-11-(tetrahydropyran-2-yloxy)-13-(tert.-butyl-dimethylsilyloxy)-14,15,16,17,18,19,20-heptanor-prostansäuremethylester

Eine Lösung von 12,3 g der nach m) erhaltenen ungesättigten Verbindung in 160 ml Essigester wurde mit 1,23 g 10 %iger Palladium-Kohle versetzt und in einer Schüttelapparatur bei Raumtemperatur und geringem Wasserstoffüberdruck hydriert. Nach Aufnahme von 640 ml Wasserstoff wurde das Lösungsmittel im Vakuum entfernt, der Rückstand in 40 ml Äther aufgenommen, die organische Phase mit gesättigter Natriumchlorid-Lösung gewaschen, über Magnesiumsulfat getrocknet und am Rotationsverdampfer zur Trockne eingeengt. Man erhielt 12,1 g der Titelverbindung.

IR (Film) : 1 740, 1 720, 1 600, 1 580, 1 275, 1 255, 1 115, 1 070, 1 025, 835, 780, 710/cm.

o) (8R,9S,11R,12S)-9-Benzoyloxy-11-(tetrahydropyran-2-yloxy)-13-hydroxy-14,15,16,17,18,19,20-heptanorprostansäuremethylester

Zu einer Lösung von 6,94 g der in der vorigen Reaktionsstufe erhaltenen Verbindung in 110 ml abs. Tetrahydrofuran wurden unter Eiskühlung 16,11 ml einer 2 m Tetrabutylammoniumfluorid-Lösung in Tetrahydrofuran gegeben. Nach 12-stündigem Rühren ner Argon-Atmosphäre wurde das Reaktionsgemisch mit 1,3 l Äther verdünnt, mit gesättigter Natriumchlorid-Lösung neutral gewaschen und die Waschlösung zweimal mit Äther nachextrahiert. Die vereinigten organischen Phasen wurden über Natriumsulfat getrocknet und im Vakuum eingeengt. Der Rückstand wurde durch Säulenchromatographie an Kieselgel mit Essigester als Fliessmittel gereinigt. Es wurden 5,54 g der gewünschten Verbindung als farbloses Öl erhalten.

IR (Film) : 3 460 (breit), 1 735, 1 715, 1 600, 1 580, 1 275, 1 115, 1 070, 1 025, 710/cm.

p) (8R,9S,11R, 12S)-9-Benzoyloxy-13-oxo-11-(tetrahydropyran-2-yloxy)-14,15,16,17,18,19,20-heptanorprostansäuremethylester

Eine Lösung von 5,54 g des nach der obigen Vorschrift erhaltenen Alkohols in 56 ml abs. Methylenchlorid wurde bei 5-10 °C innerhalb von 20 Minuten zu einer Aufschlämmung von 18,7 g Collins-Reagenz in 170 ml abs. Methylenchlorid getropft. Nach einer Stunde Nachrühren in einer Argon-Atmosphäre wurde das Reaktionsgemisch mit 500 ml eines 1/1-Gemisches aus Äther und Pentan versetzt und dekantiert. Der Kolben wurde noch zweimal mit je 500 ml des obigen Gemisches nachgewaschen. Die vereinigten organischen Phasen wurden dann dreimal mit je 50 ml 5 %iger Natriumcarbonat-Lösung, dreimal mit je 50 ml 5 %iger Schwefelsäure geschüttelt und anschliessend mit gesättigter Natriumchlorid-Lösung neutral gewaschen. Nach Trocknen über Magnesiumsulfat wurde zur Trockne eingeengt und der Rückstand über eine Kieselgel-Säule (50 g ; Fliessmittel : Essigester/Hexan = 2/8) filtriert. Man erhielt 4,6 g des gewünschten Aldehyds.

IR (Film) : 2 720, 1 735, 1 715, 1 600, 1 580, 1 275, 1 115, 1 070, 1 025, 715/cm.

q) (13E)-(8R,9S,11R,12R,16RS)-9-Benzoyloxy-16,19-dimethyl-15-oxo-11-(tetrahydropyran-2-yloxy)-13,18-prostadiensäuremethylester

Zu einer Suspension von 0,48 g 50 %igem (in Öl suspendiertem) Natriumhybrid in 60 ml frisch über Lithiumaluminiumhybrid destilliertem Dimethoxyäthan wurden unter Argon bei Raumtemperatur 2,48 g des nach Beispiel f) erhaltenen Phosphonats gelöst in 30 ml abs. Dimethoxyäthan getropft. Nach Zugabe von 0,43 g (vorher im Vakuumschrank 2 Stunden bei 50 °C getrocknet) Lithiumchlorid wurde die Reaktionsmischung zwei Stunden bei Raumtemperatur gerührt. Anschliessend wurde die Suspension auf – 20 °C abgekühlt und tropfenweise mit 4,61 g des nach p) erhaltenen Aldehyds gelöst in 90 ml abs. Dimethoxyäthan, versetzt. Danach liess man die Temperatur innerhalb von 5 Stunden von – 20 auf 0 °C und innerhalb von 1,5 Stunden weiter auf 5 °C steigen, um die Reaktionslösung dann noch drei Stunden bei Raumtemperatur zu rühren. Bei – 10 °C wurden anschliessend tropfenweise 1 ml Eisessig sowie ca. 100 ml Wasser zugegeben. Man trennte die Phasen, extrahierte die wässrige fünfmal mit Äther, vereinigte die organischen Phasen und wusch sie mit 4 %iger Natriumbicarbonat -und gesättigter Natriumchlorid-Lösung. Nach Trocknen über Magnesiumsulfat wurde am Rotationsverdampfer zur Trockne eingeengt. Der Rückstand wurde einer Reinigung durch Säulenchromatographie an Kieselgel mit Essigester/Hexan = 1/1 als Fliessmittel unterworfen. Man erhielt 5,78 g der Titelverbindung.

IR (Film) : 1 740, 1 720, 1 695, 1 670, 1 625, 1 600, 1 580, 1 270, 1 105, 1 060, 1 025, 705/cm.

r) (13E)-(8R,9S,11R,12R,15R,16RS)-9-Benzoyloxy-16,19-dimethyl-15-hydroxy-11-(tetrahydropyran-2-yloxy)-13,18-prostadiensäuremethylester

Zu einer auf – 40 °C gekühlten Lösung von 5,78 g des in der vorigen Reaktionsstufe erhaltenen Ketons in 115 ml abs. Methanol wurden portionsweise 2,37 g Natriumborhydrid gegeben. Nach einer Stunde Nachrühren bei – 40 °C wurden — ebenfalls bei dieser Temperatur — 5,09 ml Eisessig zur Reaktionslösung getropft. Nach Abziehen des Lösungsmittels am Rotationsverdampfer wurde der Rückstand mit Methylenchlorid/Wasser versetzt, die abgetrennte Wasserphase mit festem Natriumchlorid versetzt und zweimal mit Methylenchlorid extrahiert. Die vereinigten organischen Phasen wurden mit gesättigter Natriumchlorid-Lösung gewaschen, über Magnesiumsulfat getrocknet und im Vakuum eingeengt. Die Isomeren-Trennung erfolgte durch mehrfach wiederholte Säulenchromatographie an Kieselgel mit Hexan/20-100 % Essigester als Fliessmittel. Als unpolarstes Produkt wurden 2,11 g der Titelverbindung isoliert.

IR (Film) : 3 400 (breit), 1 740, 1 720, 1 600, 1 580, 1 275, 1 120, 1 030, 1 020, 710/cm.

s) (13E)-8R,9S,11R,12R,15R,16RS)-9-Benzoyloxy-16,19-dimethyl-11,15-bis-(tetrahydropyran-2-yloxy)-13,18-prostadiensäuremethylester

Zu einer Lösung von 2,11 g des nach r) erhaltenen Alkohols in 60 ml abs. Methylenchlorid gab man bei Eisbadtemperatur 0,49 ml Dihydropyran (frisch über Kaliumhydroxid destilliert) und 9,7 mg p-Toluolsulfonsäure, rührte 55 Minuten bei 0 °C und extrahierte dann die vorher mit Methylenchlorid verdünnte Reaktionslösung mit gesättigter Natriumhydrogencarbonat-Lösung und Wasser. Die organische Phase wurde über Magnesiumsulfat getrocknet und im Vakuum eingeengt. Der Rückstand wurde durch Säulenchromatographie an Kieselgel mit Essigester/Hexan = 1/2 als Fliessmittel gereinigt. Man erhielt 2,3 g der gewünschten Verbindung.

IR (Film) : 1 740, 1 720, 1 605, 1 585, 1 275, 1 115, 1 080, 1 025, 715/cm.

t) (13E)-(8R,9S,11R,12R,15R,16RS)-9-Hydroxy-16,19-dimethyl-11,15-bis-(tetrahydropyran-2-yloxy)-13,18-prostadiensäure

Eine Lösung von 2,3 g des nach s) erhaltenen Benzoats in 70 ml Methanol von 2,3 g des nach s) erhaltenen Benzoats in 70 ml Methanol wurde mit 20,6 ml 2n Kaliumhydroxid-Lösung versetzt und 31 Stunden bei Raumtemperatur gerührt. Anschliessend wurde das Reaktionsgemisch am Rotationsverdampfer eingeengt, der Rückstand in wenig Wasser aufgenommen und zweimal mit je 150 ml eines Äther/Pentan-Gemisches extrahiert. Die wässrige Phase wurde mit Zitronensäure bis pH = 5 angesäuert und dreimal mit je 150 ml Essigester extrahiert. Die vereinigten organischen Phasen wurden mit gesättigter Natriumchlorid-Lösung neutral gewaschen, über Magnesiumsulfat getrocknet und im Vakuum eingeengt. Der Rückstand wurde durch Säulenchromatographie an Kieselgel mit Hexan/50-100 % Essigester als Fliessmittel gereinigt. Es wurden 1,62 g der Titelverbindung erhalten.

IR (Film) : 3 460, 2 730, 2 660, 1 730, 1 710, 1 130, 1 110, 1 075, 1 020, 810/cm.

u) (13E)-(8R,11R,12R,15R,16RS)-9-Oxo-16,19-dimethyl-11,15-bis-(tetrahydropyran-2-Hyloxy)-13,18-prostadiensäure

Zu einer auf – 20 °C gekühlten Lösung von 360 mg des nach t) erhaltenen Alkohols in 7 ml Aceton gab man 0,46 ml Jones-Reagenz und rührte 45 Minuten bei dieser Temperatur nach. Anschliessend versetzte man mit 0,6 ml Isopropanol, rührte weitere 10 Minuten, verdünnte mit kaltem Äther, wusch dreimal mit kalter gesättigter Natriumchlorid-Lösung, trocknete die organische Phase über Magnesiumsulfat und engte im Vakuum ein. Nach Filtration über einer Sep-Pack-Fertigsäule wurden 327 mg der Titelverbindung erhalten.

IR (Film) : 2 730, 2 660, 1 740, 1 710, 1 110, 1 075, 1 025, 810/cm.

v) (13E)-(8R,11R,12R,15R,16RS)-9-Oxo-11,15-dihydroxy-16,19-dimethyl-13,18-prostadiensäure

Die nach der oben angegebenen Vorschrift erhaltenen 327 mg wurden 26 Stunden in 7 ml einer Mischung aus Eisessig/Wasser/Tetrahydrofuran (65/35/10) bei Raumtemperatur gerührt. Anschliessend engte man bei Räumtemperatur mehrere Male mit Benzol im Ölpumpenvakuum ein. Der Rückstand wurde durch Säulenchromatographie an Kieselgel mit Essigester/0-10 % Methanol als Fliessmittel gereinigt. Man erhielt 68 mg der Titelverbindung.
IR (Film) : 3 420, 2 730, 2 670, 1 735, 1 710, 1 075, 975/cm.

w) (13E)-(8R,9S,11R,12R,15S,16RS)-9-Benzoyloxy-16,19-dimethyl-15-hydroxy-11-(tetrahydropyran-2-yloxy)-13,18-prostadiensäuremethylester

Bei der Natriumborhydrid-Reduktion des Ketons nach r) wurden neben den 2,11 g β-Alkohol sowie 0,27 g α/β-Gemisch 2,67 g der Titelverbindung als polares Produkt von der Säule eluiert.
IR (Film) : 3 400 (breit), 1 740, 1 720, 1 600, 1 580, 1 275, 1 120, 1 030, 1 020, 710/cm.

x) (13E)-(8R,9S,11R,12R,15S,16RS)-9-Benzoyloxy-16,19-dimethyl-11,15-bis-(tetrahydropyran-2-yloxy)-13,18-prostadiensäuremethylester

Analog der für die Darstellung des 15-β-Isomeren in s) angegebenen Vorschrift wurden durch Umsetzung von 2,67 g des in der vorigen Reaktionsstufe angefallenen Alkohols mit 0,62 ml Dihydropyran und 12,3 mg p-Toluolsulfonsäure (Reaktionszeit : 75 Minuten) nach Säulenchromatographie an Kieselgel mit Essigester/Hexan = 1/3 als Fliessmittel 2,96 g der Titelverbindung als farbloses Öl erhalten.
IR (Film) : 1 740, 1 720, 1 600, 1 585, 1 275, 1 115, 1 075, 1 025, 715/cm.

y) (13E)-(8R-9S,11R,12R,15S,16RS)-9-Hydroxy-16,19-dimethyl-11,15-bis-(tetrahydropyran-2-yloxy)-13,18-prostadiensäure

Analog der für die Darstellung des 15 β-Isomeren in t) angegebenen Vorschrift wurde durch Umsetzung von 2,96 g des Beispiels x) mit 26,5 ml 2n Kaliumhydroxid-Lösung nach Säulenchromatographie an Kieselgel mit Hexan/50-100 % Essigester als Fliessmittel 2,22 g der Titelverbindung erhalten.
IR (Film) : 3 450, 2 730, 2 660, 1 730, 1 710, 1 130, 1 110, 1 075, 1 020, 810/cm.

z) (13E)-(8R,11R,12R,15S,16RS)-9-Oxo-16,19-dimethyl-11,15-bis-(tetrahydropyran-2-yloxy)-13,18-prostadiensäure

360 mg des in der vorigen Reaktionsstufe erhaltenen Alkohols wurden analog der für die Darstellung des entsprechenden 15 β-Isomeren in u) angegebenen Vorschrift umgesetzt. Man erhielt 326 mg der Titelverbindung.
IR (Film) : 2 730, 2 670, 1 740, 1 710, 1 105, 1 070, 1 020, 810/cm.

(13E)-(8R,11R,12R,15S,16RS)-9-Oxo-11,15-dihydroxy-16,19-dimethyl-13,18-prostadiensäure

Analog der für die Synthese des 15 β-Isomeren in v) angegebenen Vorschrift wurde durch Umsetzung von 326 mg der in der vorigen Reaktionsstufe erhaltenen Verbindung die Titelverbindung des Beispiels I) dargestellt (54 mg).
IR (Film) : 3 400, 2 730, 2 660, 1 740, 1 710, 1 075, 975/cm.

Beispiel 2

(13E)-(8R,11R,12R,15R)-11,15-Dihydroxy-16,16-19-trimethyl-9-oxo-13,18-prostadiensäure

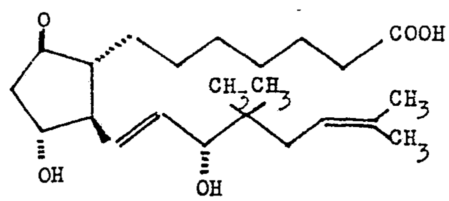

a) 2,2,5-Trimethyl-4-hexensäure-äthylester

Eine Lösung von 101,2 g Diisopropylamin in 125 ml abs. Tetrahydrofuran wurde unter Argon bei

– 20 °C tropfenweise mit 610 ml einer 1,64 n Butyllithium-Lösung in Hexan versetzt. Man ließ die Temperatur kurzzeitig auf ca. 0 °C steigen, um dann bei – 50 bis – 60 °C 116 g Isobuttersäureäthylester tropfenweise zur Lithiumdiisopropylamid-Lösung zu geben. Die Reaktionslösung wurde eine Stunde bei 0 °C gerührt, dann auf – 40 °C gekühlt und anschließend zu einer auf – 20 °C gehaltenen Lösung von 200 g 4-Brom-2-methyl-2-buten (Dimethyl-allylbromid) in 60 ml abs. Dimethylsulfoxid gegeben. Unter Ansteigenlassen der Temperatur auf Raumtemperatur wurde das Reaktionsgemisch 60 Stunden gerührt und anschließend mit 250 ml gesättigter Natriumchlorid-Lösung versetzt. Die organische Phase wurde abgetrennt und die wäßrige Phase fünfmal mit je 200 ml eines 1/1-Gemisches aus Äther und Hexan extrahiert. Die vereinigten organischen Phasen wurden mit 0,5 n Salzsäure und gesättigter Natriumchlorid-Lösung neutral gewaschen, über Magnesiumsulfat getrocknet und am Rotationsverdampfer eingeengt. Der Rückstand wurde im Vakuum destilliert. Man erhielt 91,5 g des gewünschten Esters $Kp_{13}$ = 76-81 °C.

IR (Film) : 1 735, 1 160, 1 060, 820/cm.

b) 2,2,5-Trimethyl-4-hexensäure-methylester

Die Darstellung folgte der obigen Vorschrift für die Synthese des entsprechenden Äthylesters. $Kp_{13}$ = 72-74 °C.
IR (Film) : 1 735, 1 160, 1 050, 820/cm.

c) 2,2,5-Trimethyl-4-hexensäure

Die Darstellung erfolgte nach obiger Vorschrift mit Isobuttersäure als Edukt sowie 2 Äquivalenten Lithiumdiisopropylamid. Kp 0,2-0,4 = 94-100 °C.
IR (Film) : 1 705, 1 220, 820/cm.

d) 2-(1,1,4-Trimethyl-3-pentenyl)-2-oxoäthan-phosphonsäure-dimethylester

Zu einer Lösung von 13 g Methanphosphonsäuredimethylester in 160 ml abs. Tetrahydrofuran tropfte man unter Argon bei – 60 °C 49,5 ml einer 2,02 n Butyllithium-Lösung in Hexan. Nach 15 Minuten wurde eine Lösung von 9,2 g 2,2,5-Trimethyl-4-hexensäure-äthylester in 25 ml abs. Tetrahydrofuran zugetropft. Nach 2 Stunden bei – 60 °C ließ man das Reaktionsgemisch sich innerhalb einer Stunde auf Raumtemperatur erwärmen, versetzte es mit 5,72 ml Eisessig und engte es dann im Vakuum ein. Den Rückstand, eine weiße, gelartige Masse, verteilte man in einem Zweiphasengemisch aus 35 ml Wasser und 165 ml Äther. Die organische Phase wurde über Magnesiumsulfat getrocknet und am Rotationsverdampfer eingeengt. Nach Abdestillieren der flüchtigen Nebenprodukte bei 60 °C und 0,1 Torr reinigte man den Rückstand durch Säulenchromatographie an Kieselgel mit Hexan/50-100 % Essigester als Fließmittel. Man erhielt 8,6 g der Titelverbindung.
IR (Film) : 1 705, 1 260, 1 030, 800/cm.

e) (1S,5R,6S,7R)-6-[(tert.-Butyl-dimethyl-silyloxy)-methyl]-7-benzoyloxy-2-oxabicyclo [3.3.0] octan-3-on

Zu einer Lösung von 13,8 g (1S,5R,6R,7R)-6-Hydroxymethyl-7-benzoyloxy-2-oxabicyclo [3.3.0] octan-3-on in 30 ml abs. Dimethylformamid wurden eine Lösung von 9,9 g Dimethyl-tert.-butyl-chlorsilan in 40 ml abs. Dimethylformamid und 9,35 g Imidazol gegeben. Nach zweistündigem Rühren bei Raumtemperatur und unter einer Argon-Atmosphäre zeigte die analytische Dünnschichtchromatographie vollständige Umsetzung an. Das Reaktionsgemisch wurde mit 850 ml Äther verdünnt, mit ca. 60 ml gesättigter Natriumbicarbonat-Lösung und gesättigter Natriumchlorid-Lösung gewaschen und anschliessend über Magnesiumsulfat getrocknet. Der Eindampfrückstand kann gegebenenfalls durch Säulenchromatographie an Kieselgel mit Äther als Fliessmittel gereinigt werden. Man erhielt 17,8 g der Titelverbindung (Fp = 74-75 °C nach Kristallisation aus Pentan/Äther).
IR : 1 775, 1 715, 1 600, 1 580, 1 275, 1 255, 840, 790, 720/cm.

f) (1S,5R,6S,7R)-6-[(tert.-Butyl-dimethylsilyloxy)-methyl]-7-hydroxy-2-oxabicyclo [3.3.0] octan-3-on

Eine Lösung von 17,3 g des in der vorigen Reaktionsstufe erhaltenen Benzoats in 200 ml abs. Methanol wurde bei Raumtemperatur zusammen mit 6,5 g trockenem Kaliumcarbonat unter Argon gerührt. Nach zwei Stunden zeigte die analytische Dünnschicht-Chromatographie vollständige Umsetzung an. Man tropfte daraufhin bei 0 °C 500 ml 0,1 n Salzsäure zum Reaktionsgemisch, rührte 15 Minuten bei Raumtemperatur nach, engte im Vakuum ein und extrahierte mit Essigester. Die organische Phase wurde mit gesättigter Natriumchlorid-Lösung gewaschen und über Magnesiumsulfat getrocknet.

Der Eindampfungsrückstand wurde durch Säulenchromatographie an Kieselgel mit Hexan/50-100 % Äther als Fliessmittel gereinigt. Es wurden 9,1 g der gewünschten Verbindung (Fp. : 57-58,5 °C) erhalten.
IR (Film) : 1 775, 1 255, 840, 790/cm.

g) (1S,5R,6S,7R)-6-[(tert.-Butyl-dimethylsilyloxy)-methyl]-7-(tetrahydropyran-2-yloxy)-2-oxabicyclo [3.3.0] octan-3-on

Eine Lösung von 15,8 g des nach obiger Vorschrift erhaltenen Alkohols in 300 ml destilliertem Methylenchlorid wurde zusammen mit 7,5 ml frisch über Kaliumhydroxid destilliertem Dihydropropan und 1,38 g Pyridin-p-Toluolsulfonat 14 Stunden bei Raumtemperatur gerührt. Nach dem Einengen der Reaktionslösung bei Raumtemperatur wurde mit Äther verdünnt und mit halbgesättigter Natriumchlorid-Lösung gewaschen. Die organische Lösung wurde anschliessend über Magnesiumsulfat getrocknet und dann zur Trockne eingeengt. Gegebenenfalls kann das Produkt durch Säulenchromatographie an Kieselgel mit Hexan/20-50 % Äther als Fliessmittel gereinigt werden. Die Ausbeute betrug 20 g.

IR (Film) : 1 775, 1 255, 1 115, 1 080, 1 035, 835, 775/cm.

h) (1S,3RS,5R,6S,7R)3-3-Hydroxy-6-[(tert.-Butyl-dimethylsilyloxy)-methyl]-7-(tetrahydropyran-2-yloxy)-2-oxabicyclo [3.3.0] octan

Zu einer auf − 70 °C gekühlten Lösung von 5,4 g des in der vorigen Reaktionsstufe erhaltenen Lactons in 200 ml abs. Toluol wurden unter Argon innerhalb von 15 Minuten 20 ml einer 20 %igen DIBAH-Lösung in Toluol zugetropft. Nach ca. 5 minütigem Nachrühren wurden bei der gleichen Temperatur 1,2 ml Isopropanol zugetropft bis keine Schaumbildung mehr auftrat. Man liess die Reaktionslösung sich auf 0 °C erwärmen, gab 16 ml Wasser dazu, rührte 10 Minuten nach und filtrierte über eine Fritte ab. Der Niederschlag wurde mit Essigester ausgewaschen. Die organische Phase wurde dreimal mit gesättigter Natriumchlorid-Lösung gewaschen, über Magnesiumsulfat getrocknet und am Rotationsverdampfer eingeengt. Das erhaltene Produkt (5,41 g) wurde ohne weitere Reinigung in die nächste Reaktionsstufe eingesetzt.

i) (5Z)-(8R,9S,11R,12S)-9-Hydroxy-11-(tetrahydropyran-2-yloxy)-13-(tert.-butyl-dimethyl-silyloxy)-14,14,16,17,18,19,20-heptanor-5-prostensäure

104,6 ml einer Lösung von Methansulfinylmethylnatrium in abs. DMSO (hergestellt durch Lösen von 6 g 50 % iger Natriumhydridsuspension in 120 ml abs. Dimethylsulfoxid bei maximal 70 °C) wurden bei 15 °C zu einer Lösung von 25,67 g 4-Carboxybutyl-triphenylphosphoniumbromid (vorher bei 70-80 °C an der Ölpumpe getrocknet) in 80 ml abs. Dimethylsulfoxid getropft. Diese Ylenlösung wurde 30 Minuten bei Raumtemperatur gerührt und dann unter Wasserkühlung zu einer Lösung von 5,41 g des in der vorigen Reaktionsstufe erhaltenen Lactols in 50 ml abs. Dimethylsulfoxid innerhalb von 15 Minuten getropft. Anschliessend wurde das Reaktionsgemisch vier Stunden bei 35-40 °C unter Argon gerührt. (Gegebenenfalls setzt man 50-100 ml abs. Tetrahydrofuran zur Reaktionslösung zu.)

Zur Aufarbeitung wurde das Reaktionsgemisch auf Eis/Wasser gegeben, dreimal mit Äther extrahiert, die wässrige Phase mit 10 %iger Citronensäurelösung bis pH = 4 angesäuert und dreimal mit einen 1/1-Gemisch aus Äther/Hexan extrahiert. Danach wurde noch dreimal mit Methylenchlorid ausgeschüttelt. Aufgrund der analytischen Dünnschichtchromatographie wurde die Methylenchlorid-Phase verworfen, die beiden anderen organischen Phasen dagegen vereinigt, über Magnesiumsulfat getrocknet, filtriert und am Rotationsverdampfer eingeengt. Der Rückstand wurde durch Säulenchromatographie an Kieselgel mit Hexan/70-100 % Äther als Fliessmittel gereinigt.

Man erhielt 4,32 g der Carbonsäure.

IR (Film) : 3 440 (breit), 3 200-2 500, 1 725, 1 700, 1 250, 1 100, 1 020, 830, 770/cm.

j) (5Z)-(8R,9S,11R,12S)-9-Hydroxy-11-(tetrahydropyran-2-yloxy)-13-(tert.-butyl-dimethyl-silyloxy)-14,15,16,17,18,19,20-heptanor-5-prostensäure-methylester Darstellung mit Diazomethan :

Die nach i) erhaltenen 4,32 g Carbonsäure wurden in 20 ml Methylenchlorid gelöst und so lange mit ätherischer Diazomethanlösung versetzt, bis keine Gasentwicklung mehr auftrat und die Gelbfärbung der Lösung bestehen blieb. Nach Abziehen des überschüssigen Diazomethans am Wasserstrahlvakuum wurde die Reaktionslösung am Rotationsverdampfer zur Trockne eingeengt. Man erhielt 4,3 g des gewünschten Carbonsäuremethylesters.

Darstellung mit Jodmethan :

Zu einer Lösung von 32,5 g der nach i) erhaltenen Carbonsäure in 450 ml Acetonitril wurde bei Raumtemperatur 75 ml N-Äthyldiisopropylamin und 150 ml Jodmethan in 200 ml Acetonitril innerhalb von 2 Stunden zugetropft. Es wurde eine Stunde nachgerührt, dann — nach erfolgter DC-Kontrolle — saugte man den Niederschlag ab, wusch mit Essigester nach und schüttelte die organische Phase nacheinander mit Natriumbisulfat-, Natrium-hydrogencarbonat- und Natriumchlorid-Lösung. Nach Trocknen über Magnesiumsulfat wurde am Rotationsverdampfer zur Trockne eingeengt und der Rückstand durch Säulenchromatographie an Kieselgel mit Hexan/50-100 % Äther als Laufmittel gereinigt. Man erhielt 32,3 g der Titelverbindung.

IR (Film) : 3 420 (breit), 1 740, 1 255, 1 120, 1 030, 840, 780/cm.

k) (5Z)-(8R,9S,11R,12S)-9-Benzoyloxy-11-(Tetrahydropyran-2-yloxy)-13-(tert.-butyl-dimethylsilyloxy)-14,15,16,17,18,19,20-heptanor-5-prostensäuremethylester

Zu einer Lösung von 4,7 g der nach j) erhaltenen 9-Hydroxy-Verbindung in 70 ml Pyridin wurden unter Rühren bei Raumtemperatur 2,32 ml destilliertes Benzoylchlorid getropft. Nach zweistündigem Rühren unter einer Argon-Atmosphäre wurde die Reaktionslösung mit 1,8 ml Wasser versetzt, eine weitere Stunde nachgerührt und anschliessend an der Ölpumpe bei 30 °C und 1 Torr eingeengt. Der Rückstand wurde in einem Zweiphasengemisch Äther/Wasser aufgenommen, mit Natriumbicarbonat- und gesättigter Natriumchlorid-Lösung gewaschen, über Magnesiumsulfat getrocknet und im Vakuum zur Trockne eingeengt. Nach Säulenchromatographie des Eindampfückstands an Kieselgel mit Hexan/30-50 % Essigester als Fliessmittel erhielt man 5,11 g der Titelverbindung als farbloses Öl.

IR (Film) : 1 745, 1 720, 1 605, 1 590, 1 280, 1 260, 1 120, 1 030, 840, 780, 710/cm.

l) (8R,9S,11R,12S)-9-Benzoyloxy-11-(tetrahydropyran-2-yloxy)-13-(tert.-butyl-dimethylsilyloxy)-14,15,16,17,18,19,20-heptanor-prostansäuremethylester

Eine Lösung von 12,3 g der nach k) erhaltenen ungesättigten Verbindung in 160 ml Essigester wurde mit 1,23 g 10 %iger Palladium-Kohle versetzt und in einer Schüttelapparatur bei Raumtemperatur und geringem Wasserstoffüberdruck hydriert. Nach Aufnahme von 640 ml Wasserstoff wurde das Lösungsmittel im Vakuum entfernt, der Rückstand in 40 ml Äther aufgenommen, die organische Phase mit gesättigter Natriumchlorid-Lösung gewaschen, über Magnesiumsulfat getrocknet und am Rotationsverdampfer zur Trockne eingeengt. Man erhielt 12,1 g der Titelverbindung.

IR (Film) : 1 740, 1 720, 1 600, 1 580, 1 275, 1 255, 1 115, 1 070, 1 025, 835, 780, 710/cm.

m) (8R,9S,11R,12S)-9-Benzoyloxy-11-(tetrahydropyran-2-yloxy)-13-hydroxy-14,15,16,17,18,19,20-heptanor-prostansäuremethylester

Zu einer Lösung von 6,94 g der in der vorigen Reaktionsstufe erhaltenen Verbindung in 110 ml abs. Tetrahydrofuran wurden unter Eiskühlung 16,11 ml einer 2m Tetrabutylammoniumfluorid-Lösung in Tetrahydrofuran gegeben. Nach 12-stündigem Rühren ner Argon-Atmosphäre wurde das Reaktionsgemisch mit 1,3 l Äther verdünnt, mit gesättigter Natriumchlorid-Lösung neutral gewaschen und die Waschlösung zweimal mit Äther nachextrahiert. Die vereinigten organischen Phasen wurden über Natriumsulfat getrocknet und im Vakuum eingeengt. Der Rückstand wurde durch Säulenchromatographie an Kieselgel mit Essigester als Fliessmittel gereinigt. Es wurden 5,54 g der gewünschten Verbindung als farbloses Öl erhalten.

IR (Film) : 3 460 (breit), 1 735, 1 715, 1 600, 1 580, 1 275, 1 115, 1 070, 1 025, 710/cm.

n) (8R,9S,11R,12S)-9-Benzoyloxy-13-oxo-11-(tetrahydropyran-2-yloxy)-14,15,16,17,18,19,20-heptanorprostansäuremethylester

Eine Lösung von 5,54 g des nach der obigen Vorschrift erhaltenen Alkohols in 56 ml abs. Methylenchlorid wurde bei 5-10 °C innerhalb von 20 Minuten zu einer Aufschlämmung von 18,7 g Collins-Reagenz in 170 ml abs. Methylenchlorid getropft. Nach einer Stunde Nachrühren in einer Argon-Atmosphäre wurde das Reaktionsgemisch mit 500 ml eines 1/1-Gemisches aus Äther und Pentan versetzt und dekantiert. Der Kolben wurde noch zweimal mit je 500 ml des obigen Gemisches nachgewaschen. Die vereinigten organischen Phasen wurden dann dreimal mit je 50 ml 5 %iger Natriumcarbonat-Lösung, dreimal mit je 50 ml 5 %iger Schwefelsäure geschüttelt und anschliessend mit gesättigter Natriumchlorid-Lösung neutral gewaschen. Nach Trocknen über Magnesiumsulfat wurde zur Trockne eingeengt und der Rückstand über eine Kieselgel-Säule (50 g ; Fliessmittel : Essigester/Hexan = 2/8) filtriert. Man erhielt 4,6 g des gewünschten Aldehyds.

IR (Film) : 2 720, 1 735, 1 715, 1 600, 1 580, 1 275, 1 115, 1 070, 1 025, 715/cm.

o) (13E)-(8R,9S,11R,12R)-9-Benzoyloxy-16,16,19-trimethyl-15-oxo-11-(tetrahydropyran-2-yloxy)-13,18-prostadiensäuremethylester

Zu einer Suspension von 0,74 g 50 %igem (in Öl suspendiertem) Natriumhybrid in 90 ml frisch über Lithiumaluminiumhydrid destilliertem Dimethoxyäthan wurden unter Argon bei Raumtemperatur 4,05 g des nach Beispiel d) erhaltenen Phosphonats gelöst in 45 ml abs. Dimethoxyäthan getropft. Nach Zugabe von 0,66 g (vorher im Vakuumschrank 2 Stunden bei 50 °C getrocknet) Lithiumchlorid wurde die Reaktionsmischung zwei Stunden bei Raumtemperatur gerührt. Anschliessen wurde die Suspension auf − 20 °C abgekühlt und tropfenweise mit 7,11 g des nach n) erhaltenen Aldehyds gelöst in 140 ml abs. Dimethoxyäthan, versetzt. Danach liess man die Temperatur innerhalb von 5 Stunden von − 20 auf 15 °C steigen, um die Reaktionslösung dann noch 19 Stunden bei Raumtemperatur zu rühren. Bei − 10 °C wurden anschliessend tropfenweise 1,6 ml Eisessig sowie ca. 100 ml Wasser zugegeben. Man trennte die Phasen, extrahierte die wässrige fünfmal mit Äther, vereinigte die organischen Phasen und wusch sie mit

4 %iger Natriumbicarbonat -und gesättigter Natriumchlorid-Lösung. Nach Trocknen über Magnesiumsulfat wurde am Rotationsverdampfer zur Trockne eingeengt. Der Rückstand wurde einer Reinigung durch Säulenchromatographie an Kieselgel mit Hexan 150-100 % Essigester als Fließmittel unterworfen. Man erhielt 9,19 g der Titelverbindung.

IR (Film) : 1 740, 1 720, 1 695, 1 670, 1 625, 1 600, 1 580, 1 270, 1 105, 1 060, 1 030, 705/cm.

p) (13E)-(8R,9S,11R,12R,15S)-9-Benzoyloxy-16,16,19-trimethyl-15-hydroxy-11-(tetrahydropyran-2-yloxy)-13,18-prostadiensäuremethylester

Zu einer auf − 40 °C gekühlten Lösung von 9,19 g des in der vorigen Reaktionsstufe erhaltenen Ketons in 180 ml abs. Methanol wurden portionsweise 3,68 g Natriumborhydrid gegeben. Nach 3,5 Stunden Nachrühren bei − 40°C wurden — ebenfalls bei dieser Temperatur — 7,9 ml Eisessig zur Reaktionslösung getropft. Nach Abziehen des Lösungsmittels am Rotationsverdampfer wurde der Rückstand mit Methylenchlorid/Wasser versetzt, die abgetrennte Wasserphase mit festem Natriumchlorid versetzt und zweimal mit Methylenchlorid extrahiert. Die vereinigten organischen Phasen wurden mit gesättigter Natriumchlorid-Lösung gewaschen, über Magnesiumsulfat getrocknet und im Vakuum eingeengt. Die Isomeren-Trennung erfolgte durch mehrfach wiederholte Säulenchromatographie an Kieselgel mit Hexan/20-40 % Essigester als Fliessmittel. Als unpolarstes Produkt wurden 2,02 g der Titelverbindung isoliert.

IR (Film) : 3 400 (breit), 1 740, 1 720, 1 600, 1 580, 1 275, 1 120, 1 030, 1 025, 710/cm.

q) (13E)-8R,9S,11R,12R-15S)-9-Benzoyloxy-16,16,19-trimethyl-11,15-bis-(tetrahydropyran-2-yloxy)-13,18-prostadiensäuremethylester

Zu einer Lösung von 2 g des nach p) erhaltenen Alkohols in 60 ml abs. Methylenchlorid gab man bei Eisbadtemperatur 0,45 ml Dihydropyran (frisch über Kaliumhydroxid destilliert) und 9 mg p-Toluolsulfonsäure, rührte 55 Minuten bei 0 °C und extrahierte dann die vorher mit Methylenchlorid verdünnte Reaktionslösung mit gesättigter Natriumhydrogencarbonat-Lösung und Wasser. Die organische Phase wurde über Magnesiumsulfat getrocknet und im Vakuum eingeengt. Der Rückstand wurde durch Säulenchromatographie an Kieselgel mit Essigester/Hexan = 1/2 als Fliessmittel gereinigt. Man erhielt 2,12 g der gewünschten Verbindung.

IR (Film) : 1 740, 1 720, 1 605, 1 585, 1 275, 1 115, 1 080, 1 020, 715/cm.

r) (13E)-(8R,9S,11R,12R,15S)-9-Hydroxy-16,16,19-trimethyl-11,15-bis-(tetrahydropyran-2-yloxy)-13, 18-prostadiensäure

Eine Lösung von 2,12 g des nach q) erhaltenen Benzoats in 60 ml Methanol wurde mit 19 ml 2n Kaliumhydroxid-Lösung versetzt und 26 Stunden bei Raumtemperatur gerührt. Anschliessend wurde das Reaktionsgemisch am Rotationsverdampfer eingeengt, der Rückstand in wenig Wasser aufgenommen und zweimal mit je 150 ml eines Äther/Pentan-Gemisches extrahiert. Die wässrige Phase wurde mit Zitronensäure bis pH = 5 angesäuert und dreimal mit je 150 ml Essigester extrahiert. Die vereinigten organischen Phasen wurden mit gesättigter Natriumchlorid-Lösung neutral gewaschen, über Magnesiumsulfat getrocknet und im Vakuum eingeengt. Der Rückstand wurde durch Säulenchromatographie an Kieselgel mit Hexan/50-100 % Essigester als Fliessmittel gereinigt. Es wurden 1,43 g der Titelverbindung erhalten.

IR (Film) : 3 460, 2 730, 2 660, 1 730, 1 710, 1 130, 1 110, 1 075, 1 025, 810/cm.

s) (13E)-(8R,11R,12R,15S)-9-Oxo-16,16,19-trimethyl-11,15-bis-(tetrahydropyran-2-yloxy)-13,18-prostadiensäure

Zu einer auf − 20 °C gekülten Lösung von 500 mg des nachr r) erhaltenen Alkohols in 10 ml Aceton gab man 0,63 ml Jones-Reagenz und rührte 45 Minuten bei dieser Temperatur nach. Anschliessend versetzte man mit 0,8 ml Isopropanol, rührte weitere 10 Minuten, verdünnte mit kaltem Äther, wusch dreimal mit kalter gesättigter Natriumchlorid-Lösung, trocknete die organische Phase über Magnesiumsulfat und engte im Vakuum ein. Nach Filtration über einer Sep-Pack-Fertigsäule wurden 442 mg erhalten.

IR (Film) : 2 730, 2 660, 1 740, 1 710, 1 105, 1 070, 1 025, 810/cm.

t) (13E)-(8R,11R,12R,15S)-11,15-Dihydroxy-16,16,19-trimethyl-9-oxo-13,18-prostadiensäure

Die nach der oben angegebenen Vorschrift erhaltenen 442 mg wurden 24 Stunden in 9 ml einer Mischung aus Eisessig/Wasser/Tetrahydrofuran (65/35/10) bei Raumtemperatur gerührt. Anschliessend engte man bei Räumtemperatur mehrere Male mit Benzol im Ölpumpenvakuum ein. Der Rückstand wurde durch Säulenchromatographie an Kieselgel mit Essigester/0-10 % Methanol als Fliessmittel gereinigt. Man erhielt 124 mg der Titelverbindung.

IR (Film) : 3 420, 2 730, 2 670, 1 740, 1 710, 1 075, 975/cm.

u) (13E)-(8R,9S,11R,12R,15R)-8-Benzoyloxy-16,16,19-trimethyl-15-hydroxy-11-(tetrahydropyran-2-yloxy)-13,18-prostadiensäuremethylester

Bei der Natriumborhydrid-Reduktion des Ketons nach p) wurden neben den 2,02 g β-Alkohol sowie 0,7 g α/β-Gemisch 4,28 g der Titelverbindung als polares Produkt von der Säule eluiert.

IR (Film) : 3 400 (breit), 1 740, 1 720, 1 600, 1 575, 1 275, 1 120, 1 030, 1 020, 710/cm.

v) (13E)-(8R,9S,11R,12R,15R)-9-Benzoyloxy-16,16,19-trimethyl-11,15-bis-(tetrahydropyran-2-yloxy)-13,18-prostadiensäuremethylester

Analog der für die Darstellung des 15-β-Isomeren in q) angegebenen Vorschrift wurden durch Umsetzung von 4,28 g des in der vorigen Reaktionsstufe angefallenen Alkohols mit 0,96 ml Dihydropyran und 11 mg p-Toluolsulfonsäure (Reaktionszeit : 75 Minuten) nach Säulenchromatographie an Kieselgel mit Essigester/Hexan = 1/3 als Fliessmittel 4,3 g der Titelverbindung als farbloses Öl erhalten.

IR (Film) : 1 740, 1 720, 1 600, 1 585, 1 275, 1 115, 1 075, 1 020, 715/cm.

w) (13E)-(8R,9S,11R,12R,15R)-9-Hydroxy-16,16,19-trimethyl-11,15-bis-(tetrahydropyran-2-yloxy)-13,18-prostadiensäure

Analog der für die Darstellung des 15 β-Isomeren in r) angegebenen Vorschrift wurde durch Umsetzung von 4,3 g des Beispiels v) mit 38,5 ml 2n Kaliumhydroxid-Lösung nach Säulenchromatographie an Kieselgel mit Hexan/50-100 % Essigester als Fliessmittel 2,8 g der Titelverbindung erhalten.

IR (Film) : 3 450, 2 730, 2 660, 1 730, 1 710, 1 130, 1 110, 1 075, 1 025, 810/cm.

x) (13E)-(8R,11R,12R,15R)-9-Oxo-16,16-19-trimethyl-11,15-bis-(tetrahydropyran-2-yloxy)-13,18-prostadiensäure

500 mg des in der vorigen Reaktionsstufe erhaltenen Alkohols wurden analog der für die Darstellung des entsprechenden 15 β-Isomeren in s) angegebenen Vorschrift umgesetzt. Man erhielt 410 mg der Titelverbindung

IR (Film) : 2 730, 2 670, 1 740, 1 710, 1 110, 1 075, 1 020, 810/cm.

y) (13E)-(8R,11R,12R,15R)-11,15-Dihydroxy-16,16,19-trimethyl-9-oxo-13,18-prostadiensäure

Analog der für die Synthese des 15 β-Isomeren in x) angegebenen Vorschrift wurde durch Umsetzung von 410 mg der in der vorigen Reaktionsstufe erhaltenen Verbindung die Titelverbindung erhalten (110 mg).

IR (Film) : 3 400, 2 730, 2 660, 1 740, 1 710, 1 075, 975/cm.

Beispiel 3

(13E)-(8R,11R,12R,15S,16RS)-11,15-Dihydroxy-16,19-dimethyl-9-oxo-13,18-prostadiensäure, Tris(hydroxymethyl)-aminomethan-Salz.

Zu einer Lösung von 94,5 mg der Verbindung aus Beispiel 1 in 14 ml Acetonitril wurde bei 60 °C eine Lösung von 32,9 mg Tris(hydroxymethyl)-aminomethan in 0,1 ml Wasser zugesetzt und 14 Stunden bei Raumtemperatur stehengelassen. Ausbeute 63,5 mg.

Beispiel 4

(13E)-(8R,11R,12R,15R)-11,15-Dihydroxy-16,16,19-trimethyl-9-oxo-13,18-prostadiensäure, Tris(hydroxymethyl)-aminomethan-Salz.

Zu einer Lösung von 98 mg der Verbindung aus Beispiel 2 in 14 ml Acetonitril wurde bei 60 °C eine Lösung von 32,9 mg Tris(hydroxymethyl)-aminomethan in 0,1 ml Wasser zugesetzt und 14 Stunden bei Raumtemperatur stehengelassen. Ausbeute 68,5 mg.

Beispiel 5

Zusammensetzung einer Ampulle für Injektionszwecke.

0,5 mg (13E)-(8R,11R,12R,15S,16RS)-11,15-Dihydroxy-16,19-dimethyl-9-oxo-13,18-prostadiensäure
8,9 mg NaCl
1,212 mg Trometamol
0,01 ml Ethanol (96 %)
ad 1 ml mit Wasser für Injektionszwecke.

**Ansprüche**

1. (13E)-(8R,11R,12R)-11,15-Dihydroxy-16,19-dimethyl-9-oxo-13,18-prostadiensäuren der allgemeinen Formel I

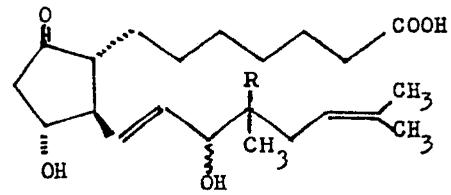

(I)

worin

R Wasserstoff oder eine Methylgruppe bedeutet oder ihre physiologisch verträglichen Salze mit Basen

2. (13E)-(8R,11R,12R,15S,16RS)-11,15-Dihydroxy-16,19-dimethyl-9-oxo-13,18-prostadiensäure

3. (13E)-(8R,11R,12R,15R)-11,15-Dihydroxy-16,16,19-trimethyl-9-oxo-13,18-prostadiensäure

4. (13E)-(8R-11R,12R,15S,16RS)-11,15-Dihydroxy-16,19-dimethyl-9-oxo-13,18-prostadiensäure, Tris(hydroxymethyl)aminomethan-Salz

5. (13E)-(8R,11R,12R,15R)-11,15-Dihydroxy-16,16-19-trimethyl-9-oxo-13,18-prostadiensäure, Tris(hydroxymethyl)aminomethan-Salz

6. Verfahren zur Herstellung von Verbindungen der Formel I, dadurch gekennzeichnet, daß man (8R,9S,11R,12S)-9-Benzoyloxy-13-oxo-11-(tetrahydropyran-2-yloxy)-14,15,16,17,18,19,20-heptanorprostansäuremethylester mit 2-(1,4-Dimethyl-3-pentenyl)-2-oxoäthanphosphonsäuredimethylestem der Formel II

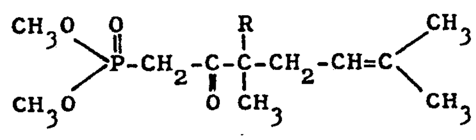

(II)

worin

R die oben angegebene Bedeutung hat, umsetzt und anschließend die 15-Ketogruppe reduziert und mit Dihydropyran schützt, die 9-Hydroxygruppe nach Freisetzung oxidiert, vorhandene Schutzgruppen abspaltet, wenn R Wasserstoff bedeutet, in die Stereoisomeren trennt und gegebenenfalls in ein physiologisch verträgliches Salz überführt.

7. Pharmazeutische Zusammensetzungen, dadurch gekennzeichnet, daß sie als aktiven Bestandteil eine Verbindung aus Anspruch 1 zusammen mit einem oder mehreren pharmazeutischen Trägern oder Verdünnungsmitteln enthalten.

**Claims**

1. (13E)-(8R,11R,12R)-11,15-dihydroxy-16,19-dimethyl-9-oxo-13,18-prostadienoic acids of the general formula I

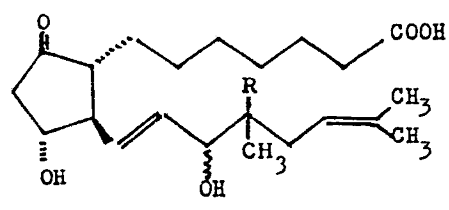

(I)

in which

R represents hydrogen or a methyl group, or the physiologically tolerable salts thereof with bases.

2. (13E)-(8R,11R,12R,15S,16RS)-11,15-dihydroxy-16,19-dimethyl-9-oxo-13,18-prostadienoic acid.

3. (13E)-(8R,11R,12R,15R)-11,15-dihydroxy-16,16,19-trimethyl-9-oxo-13,18-prostadienoic acid.

4. (13E)-(8R,11R,12R,15S,16RS)-11,15-dihydroxy-16,19-dimethyl-9-oxo-13,18-prostadienoic acid, tris(hydroxymethyl) aminomethane salt.

5. (13E)-(8R,11R,12R,15R)-11,15-dihydroxy-16,16,19-trimethyl-9-oxo-13,18-prostadienoic acid, tris(hydroxymethyl)aminomethane salt.

6. Process for the manufacture of compounds of the formula I, characterised in that (8R,9S,11R,12S)-9-benzoyloxy-13-oxo-11-(tetrahydropyran-2-yloxy)-14,15,16,17,18,19,20-heptanorprostanoic acid methyl ester is reacted with 2-(1,4-dimethyl-3-pentenyl)-2-oxoethanephosphonic acid dimethyl esters of the formula II

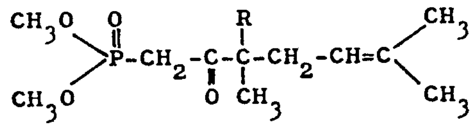

(II)

in which

R has the meaning given above, and subsequently the 15-keto group is reduced and protected with dihydropyran, the 9-hydroxy group is liberated and then oxidised, protecting groups present are split off, if R represents hydrogen the compound is separated into its stereoisomers, and, optionally, the compound is converted into a physiologically tolerable salt.

7. Pharmaceutical compositions, characterised in that they contain as active component a compound from claim 1 together with one or more pharmaceutical carriers or diluents.

**Revendications**

1. Acides dihydroxy-11,15 diméthyl-16,19 oxo-9 prostadiène-13,18 oïques-(13E)-(8R,11R,12R) répondant à la formule générale I

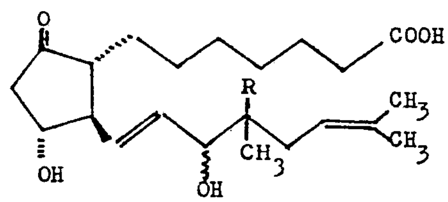

(I)

dans laquelle

R représente un atome d'hydrogène ou un radical méthyle, ainsi que les sels acceptables du point de vue physiologique qu'ils forment avec des bases.

2. Acide dihydroxy-11,15 diméthyl-16,19 oxo-9 prostadiène-13,18 oïque-(13E)-(8R,11R,12R,15S, 16RS).

3. Acide dihydroxy-11,15 triméthyl-16,16,19 oxo-9 prostadiène-13,18 oïque-(13E)-(8R,11R,12R,15R).

4. Sel de tris-(hydroxyméthyl)-aminométhane de l'acide dihydroxy-11,15 diméthyl-16,19 oxo-9 prostadiène-13,18 oïque-(13E)-(8R,11R,12R,15S,16RS).

5. Sel de tris-(hydroxyméthyl)-aminométhane de l'acide dihydroxy-11,15 triméthyl-16,16,19 oxo-9 prostadiène-13,18 oïque-(13E)-(8R,11R,12R,15R).

6. Procédé de préparation de composés de formule I, procédé caractérisé en ce qu'on fait réagir l'ester méthylique de l'acide benzoyloxy-9 oxo-13 (tétrahydropyrannyl-2 oxy)-11 heptanor-14,15,16,17,18, 19,20 prostanoïque-(8R,9S,11R,12S) avec un oxo-2 diméthyl-3,6 heptène-5 phosphonate-1 de diméthyle répondant à la formule II :

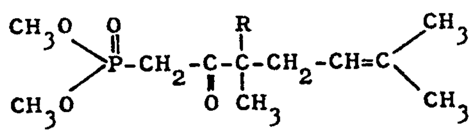

(II)

dans laquelle

R a la signification précédemment donnée, puis on réduit le radical oxo en 15 et on protège au moyen du dihydropyranne, on oxyde le radical hydroxy en 9 après l'avoir libéré, on coupe les éventuels radicaux protecteurs, on fractionne le produit en les stéréo-isomères dans le cas où R représente l'hydrogène, et, le cas échéant, on transforme le composé obtenu en un sel acceptable du point de vue physiologique.

7. Médicaments caractérisés en ce qu'ils contiennent, comme substance active, un composé selon la revendication 1, associé à un ou plusieurs excipients ou diluants pharmaceutiques.